# EUROPEAN PATENT SPECIFICATION

(11) **EP 1 513 528 B1**
(45) Date of publication and mention of the grant of the patent: **20.05.2009**
(21) Application number: 03741747.4
(22) Date of filing: 18.06.2003
(51) Int. Cl.: A61K 31/445, A61P 25/28

(54) **PHARMACEUTICAL COMPOSITION CONTAINING STABILISED AMORPHOUS FORM OF DONEPEZIL HYDROCHLORIDE**
PHARMAZEUTISCHE ZUSAMMENSETZUNG ENTHALTEND STABILISIERTE AMORPHE FORM VON DONEPEZIL HYDROCHLORID
COMPOSITION PHARMACEUTIQUE CONTENANT UNE FORME AMORPHE STABILISEE D'HYDROCHLORURE DE DONEPEZIL

(30) Priority: 19.06.2002 SI 200200155
(43) Date of publication of application: 16.03.2005
(73) Proprietor: Krka Tovarna Zdravil, D.D., Novo Mesto, 8501 Novo Mesto (SI)
(72) Inventor: VRBINC, Miha, 8000 Novo mesto (SI); PUNCUH KOLAR, Alesa, 8311 Kostanjevica na Krki (SI); VRECER, Franc, 8351 Straza pri Novem mestu (SI); KOTAR JORDAN, Berta, 8311 Kostanjevica na Krki (SI); STANGELJ, Veronika, 8322 Stopice (SI)
(74) Representative: UEXKÜLL & STOLBERG
(86) International application number: PCT/SI2003/000019
(87) International publication number: WO 2004/000317

(56) References cited:
- EP-A- 1 027 887
- WO-A-01/34119
- WO-A-97/46527
- US-A- 4 610 875
- BRYSON H M ET AL: "DONEPEZIL" DRUGS AND AGING, ADIS INTERNATIONAL LTD, NZ, vol. 10, no. 3, 1997, pages 234-239, XP000900063 ISSN: 1170-229X

## Description

The present invention relates to a stable pharmaceutical composition containing 1-benzyl-4-((5,6-dimethoxy-1-indanon)-2-yl)methyl-piperidine (donepezil) hydrochloride and to a method for the stabilisation of this compound.

### Technical Field of the Invention

Donepezil hydrochloride is a well-known medicinal active ingredient with a strong and highly selective acetylcholinesterase inhibition and is identified by the following chemical formula:

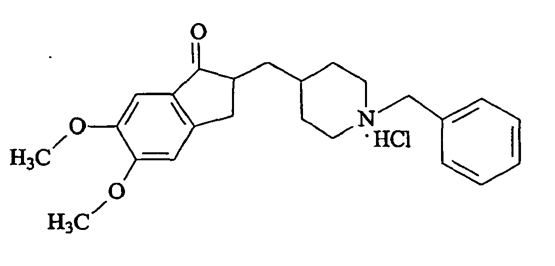

Donepezil is described to be effective in the treatment and/or prevention of various diseases such as senile dementia, particularly senile dementia of Alzheimer's type, cerebrovascular diseases accompanying cerebral apoplexy, e.g. cerebral haemorrhages or cerebral infarct, cerebral arteriosclerosis, head injuries and other diseases. It is also used in the treatment and/or prevention of aprosexia, speech disorders, hypobulia, mood changes, attention deficit hyperreactivity disorders, recent memory disturbances, hallucinatory-paranoid syndrome, behaviour changes and other similar diseases accompanying encephalitis, cerebral palsy and others.

### Prior Art

Various processes for the synthesis of donepezil and/or compositions with donepezil as a medicinal active ingredient have been described.

WO 97/46527 describes a method for the preparation of polymorphs I-V and of an amorphous form of donepezil hydrochloride. The amorphous form is prepared in such a way that donepezil hydrochloride is dissolved in water, the solution is cooled to a low temperature and freeze-dried for 4 days at the temperature -82°C. When comparing the results of stability testing it was established that the polymorphs I-IV are preferred over the amorphous form because they show enhanced chemical stability. Thus, the amorphous form is less stable when compared with the polymorphs I-IV. After a three-week exposal to the temperature of 80°C, the content of impurities rose from 0.12% at time 0 (zero) to 3.29%. Further, in hygroscopicity testing it was found that the amorphous form is significantly more hygroscopic when exposed to different relative humidities as compared to polymorphs I-IV. At the exposure of the amorphous form to a 100.0 % relative humidity, the water content rose from initial 2.03% to 15.44%.

EP-A-1 086 706 describes the preparation of donepezil in salt form to enhance stability. In order to achieve a higher stability the use of organic acids is suggested to be preferred over hydrochloric acid, since the use of the latter yields a higher amount of impurities. When 5 mg of hydrochloric acid were added to 5 mg/5 ml of donepezil solution and it was stored at 60°C for one month, the ratio of the area of impurities on the chromatogram was 0.42%. In a second experiment, wherein 5 mg of hydrochloric acid were added to 5 mg/5 ml of donepezil solution containing 10% of povidone and stored at the same conditions (60°C, one month), the ratio of the area of impurities on a chromatogram was 2.45%.

EP-A-1 120 109 describes the use of steam-extruded polymers for enhancing the disintegration and dissolution of a pharmaceutical composition. Although an amorphous form is described in this document, the rapid disintegration was achieved by the use of steam-extruded polymers.

According to the literature data, amorphous forms are generally more hygroscopic and less stable than crystalline forms and crystallize into a crystalline form or a mixture of crystalline forms (Brittain HG. Polymorphism in Pharmaceutical Solids. 1st ed. New York: Marcel Dekker, 1999). Amorphous substances may be stabilised by the addition of crystallization inhibitors (Chem. Pharm. Bull. 1983; 31 (7): 2510-2512).

EP-A-1 027 887 (PFIZER PROD INC) 16 August 2000 discloses the principle of stabilizing drugs in the amorphous state by the addition of certain polymers. The formulations of EP-A-1 027 887 are produced by dissolving the drug and the polymer in a solvent followed by spray-drying or spray-coating.

### Technical Problem

Considering all the facts described in the prior art, there existed a need to prepare a stable formulation containing the amorphous form of donepezil hydrochloride. This problem was solved by preparing donepezil hydrochloride *in situ* and stabilizing the amorphous form by the addition of a crystallization inhibitor.

### Detailed Description of the Invention

An object of the present invention is a pharmaceutical composition containing amorphous or stabilised amorphous donepezil hydrochloride, which composition is obtained by preparing donepezil hydrochloride *in situ* and by adding a crystallization inhibitor.

A process for the preparation of a pharmaceutical composition containing donepezil hydrochloride comprises the following steps:
a) dispersing donepezil in a pharmaceutically acceptable solvent and adding a hydrochloric acid solution to the dispersion,
   or *vice versa,* adding donepezil to a hydrochloric acid solution in a pharmaceutically acceptable solvent, and
b) loading the obtained solution onto a mixture of pharmaceutically acceptable carriers.

The crystallization inhibitor/s can be added either to the solution obtained under a) or to the mixture of pharmaceutically acceptable carriers under b).

A further object of the present invention is the preparation of the amorphous form of donepezil hydrochloride by spray-drying a donepezil hydrochloride solution. The solution of donepezil hydrochloride can be prepared either *in situ* by adding a hydrochloric acid solution to a dispersion of donepezil, or *vice versa,* by dissolving donepezil in hydrochloric acid or by dissolving donepezil hydrochloride in a pharmaceutically acceptable solvent or a mixture of solvents.

Optionally, the amorphous form of donepezil hydrochloride can be stabilised by dissolving a crystallization inhibitor in a solution of donepezil hydrochloride, whereupon the solvent is removed by spray-drying. Subsequently, the amorphous form or the stabilised amorphous form of donepezil hydrochloride can be dissolved in a pharmaceutically acceptable solvent and further processed by loading the obtained solution onto a mixture of pharmaceutically acceptable excipients. The dry amorphous form or the stabilised dry amorphous form of donepezil hydrochloride can be admixed to pharmaceutically acceptable excipients so as to prepare a pharmaceutical composition.

The solution of donepezil hydrochloride prepared *in situ* or by dissolving the amorphous form or the stabilised amorphous form of donepezil hydrochloride, can be loaded onto pharmaceutically acceptable carriers such as a powdered mixture of excipients, pellets, crystals, granules (e.g. granulated excipients), microtablets, tablets.

The final pharmaceutical dosage forms can be formulated by known methods such as wet or dry granulation, direct tabletting, pelletisation.

The conventional equipment used for manufacturing pharmaceutical compositions can be a high shear mixer/granulator, Wurster coating system, fluid bed granulator (top spray, bottom spray, tangential spray), conventional coating pans etc.

The pharmaceutical composition can be in any conventional pharmaceutical unit dosage form such as granules, pellets, tablets, film-coated tablets, fast dispersible tablets, capsules etc.

According to the present invention, the crystallization inhibitor is selected from at least one of cellulose derivatives, polyvinylpyrrolidone and derivatives thereof, xanthan gums, pectins, alginates, tragacanth and derivatives, gum arabic and derivatives, carrageenans, agar and derivatives, polysaccharides from microbiological sources, arabinogalactanes, galactomannans, dextrans and the like. The preferred crystallization inhibitors are cellulose derivatives and polyvinylpyrrolidone and its derivatives.

The crystallization inhibitor is added in an amount from 0.0001 g to 5.0 g for 1 g of donepezil hydrochloride, preferably between 0.5 g to 2.0 g.

The solution is prepared by the addition of donepezil to hydrochloric acid in a molar ratio of 1 : 0.5-5, preferably in a molar ratio of 1:0.5-3. The pH value of the solution obtained is between 2 and 6, preferably between 3.0 to 5.5.

The pharmaceutically acceptable solvents are water, a mixture of water with water-miscible solvents, acetone, alcohols such as ethanol, methanol, isopropanol, preferably water or a mixture of water with alcohols, or alcohols.

The pharmaceutically acceptable excipients can be diluents such as microcrystalline cellulose, lactose (anhydrous or in monohydrate form), other sugars such as mannitol, saccharose or a mixture thereof, siliconised microcrystalline cellulose, calcium hydrogen phosphate; binders such as povidone, microcrystalline cellulose, hydroxyethylcellulose, hydroxypropylcellulose, hypromellose, starch, pregelatinised starch; disintegrants such as maize starch, sodium starch glycolate, crospovidone, microcrystalline cellulose, carboxymethylcellulose sodium, Amberlite^{®}; lubricants such as magnesium stearate, calcium stearate, sodium laurylsulphate, hydrogenated vegetable oil, hydrogenated castor oil, sodium stearyl fumarate.

The method according to the present invention is advantageous over the known prior art in that, unexpectedly and surprisingly, a highly stable amorphous form of donepezil hydrochloride with a low content of impurities in the pharmaceutical composition is obtained. The results of the stability testing of the pharmaceutical compositions with the amorphous form according to the present invention are presented in Table 1.

**Table 1**

| Time of exposure to air at 40°C/75% RH; (days) | Total amount of related substances (%) |
|---|---|
| 0 | 0.05 |
| 28 | 0.09 |

The present invention is illustrated by the following Examples without being limited thereto.

### Example 1

### Preparation of tablets

Donepezil (20 g) was dispersed in a pre-prepared aqueous solution (240 ml) containing concentrated hydrochloric acid (4 ml) and povidone (20 g). Lactose monohydrate (294 g), microcrystalline cellulose (200 g), maize starch (30 g) and hydroxyethylcellulose (15 g) were passed through a 30-mesh sieve and subsequently mixed in a fluid bed granulator.

The aqueous solution of donepezil hydrochloride to be granulated was sprayed onto the powder mixture in the fluid-bed granulator and after the completed spraying the obtained granulate was dried in a fluid-bed dryer at an inlet temperature of 60°C. The dried granulate was passed through an 18-mesh sieve and mixed with maize starch (30 g) and magnesium stearate (3 g) in a biconic mixer.

The mixture to be tabletted (the granulate with added maize starch and magnesium stearate) was compressed into tablets, each containing 5 mg of donepezil hydrochloride. The average weight of the tablets was 140 mg.

### Example 2

### Preparation of tablets

Donepezil (20 g) was dispersed in a pre-prepared aqueous solution (240 ml) containing 0.5 M hydrochloric acid (35 ml). The thus prepared aqueous solution of donepezil hydrochloride was admixed with an aqueous solution containing hypromellose (10 g). Microcrystalline cellulose (262 g), lactose monohydrate (262 g) and maize starch (25 g) were passed through a 30-mesh sieve and subsequently mixed in a fluid-bed granulator.

The aqueous solution of donepezil hydrochloride was sprayed onto the powder mixture in the fluid-bed granulator and the obtained granulate was then dried in a fluid-bed dryer at an inlet temperature of 70°C. The dried granulate was passed through an 18-mesh sieve and mixed with maize starch (30 g) and hydrogenated vegetable oil (3 g) in a biconic mixer.

The mixture to be tabletted (the granulate with admixed maize starch and hydrogenated castor oil) was compressed into tablets, each containing 5 mg of donepezil hydrochloride. The average weight of the tablets was 140 mg.

### Example 3

### Preparation of tablets

Donepezil (30 g) was dispersed in purified water (320 g). During a fast stirring of the dispersion, concentrated hydrochloric acid (6 ml) was slowly added. A mixture of lactose monohydrate (818 g), crospovidone (40 g) and povidone (25 g) was passed through a 30-mesh sieve and subsequently mixed in a fluid-bed granulator.

The aqueous solution of donepezil hydrochloride to be granulated was sprayed onto the powder mixture in the fluid-bed granulator and the obtained granulate was then dried in a fluid-bed dryer at an inlet temperature of 60°C. The dried granulate was passed through an 18-mesh sieve and mixed with magnesium stearate (5 g) in a biconic mixer.

The mixture to be tabletted (the granulate with admixed magnesium stearate) was compressed into tablets, each containing 10 mg of donepezil hydrochloride. The average weight of the tablets was 280 mg.

### Example 4

### Preparation of cores

Donepezil (34 g) was dispersed in purified water (360 g). During a fast stirring of the dispersion, concentrated hydrochloric acid (7 ml) was slowly added. Lactose monohydrate (605 g), microcrystalline cellulose (231 g), maize starch (50 g) and hydroxypropylcellulose (30 g) were passed through a 30-mesh sieve and subsequently mixed in a fluid-bed granulator.

The aqueous solution of donepezil hydrochloride (pH = 4.5) to be granulated was sprayed onto the powder mixture in the fluid-bed granulator and the obtained granulate was then dried in a fluid-bed dryer at an inlet temperature of 70°C. The dried granulate was passed through an 18-mesh sieve and admixed with maize starch (50 g) and magnesium stearate (5 g) in a biconic mixer.

The mixture to be tabletted (the granulate with admixed maize starch and magnesium stearate) was compressed into cores, each containing 10 mg of donepezil hydrochloride. The average weight of the cores was 280 mg.

### Preparation of film-coated tablets

The obtained cores were coated with a conventional ready-to-make mixture for the preparation of a film-coating suspension containing hydroxypropylmethylcellulose, macrogol 400, iron oxide yellow and titanium dioxide, until the average weight of the film coated tablets was 288 mg.

The pH value of film coated tablets dispersed in purified water was 6.4.

### Example 5

### Preparation of tablets

Donepezil (300 g) was dispersed in purified water (300 g). During a fast stirring of the dispersion 0.5 M hydrochloric acid (520 ml) was slowly added. The aqueous solution of donepezil hydrochloride was spray-dried on a mini spray-dryer Büchi 190.

Amorphous donepezil hydrochloride (100 g) was dissolved in purified water (1000 ml). Hydroxypropylcellulose (85 g), lactose monohydrate (1680 g), microcrystalline cellulose (640 g) and maize starch (140 g) were passed through a 30-mesh sieve.

The aqueous solution of donepezil hydrochloride to be granulated was sprayed onto the powder mixture in a fluid-bed granulator and the obtained granulate was then dried in a fluid-bed dryer at an inlet temperature of 65°C. The dried granulate was passed through an 18-mesh sieve and admixed with maize starch (140 g) and magnesium stearate (15 g) in a biconic mixer.

The mixture to be tabletted (the granulate with admixed maize starch and magnesium stearate) was compressed into tablets, each containing 5 mg of donepezil hydrochloride. The average weight of the tablets was 140 mg.

### Example 6

### Preparation of cores

Donepezil (300 g) was dispersed in purified water (300 g). During a fast stirring of the dispersion, 0.5 M hydrochloric acid (520 ml) was slowly added. Separately, povidone (329 g) was dissolved in purified water (1200 ml). Both solutions were mixed together in such a manner that the aqueous solution of donepezil hydrochloride was added to the solution of povidone. The aqueous solution obtained was spray-dried on a mini spray-dryer Büchi 190.

Amorphous donepezil hydrochloride (50 g) stabilised with povidone (50 g) was dissolved in purified water (1000 ml). Hydroxypropylcellulose (42 g), lactose monohydrate (790 g), microcrystalline cellulose (320 g) and maize starch (70 g) were passed through a 30-mesh sieve.

The aqueous solution of donepezil hydrochloride to be granulated was sprayed onto the powder mixture in a fluid-bed granulator and the obtained granulate was then dried in a fluid-bed dryer at an inlet temperature of 65°C. The dried granulate was passed through an 18-mesh sieve and admixed with maize starch (70 g) and magnesium stearate (8 g) in a biconic mixer.

The mixture to be tabletted (the granulate with admixed maize starch and magnesium stearate) was compressed into cores, each containing 5 mg of donepezil hydrochloride. The average weight of the cores was 140 mg.

### Preparation of film coated tablets

The obtained cores were coated with a conventional ready-to-make mixture for the preparation of a film-coating suspension containing hydroxypropylmethylcellulose, macrogol 400 and titanium dioxide, until the average weight of the film coated tablets was 144 mg.

## Claims

1. Pharmaceutical composition with donepezil hydrochloride containing an amorphous form or stabilised amorphous form thereof, **characterized in that** it is obtainable by preparing donepezil hydrochloride *in situ* and by adding a crystallization inhibitor.

2. Pharmaceutical composition according to claim 1, **characterized in that** the crystallization inhibitor is selected from at least one of cellulose derivatives, polyvinylpyrrolidone and derivatives thereof, xanthan gums, pectins, alginates, tragacanth and derivatives, gum arabic and derivatives, carrageenans, agar and derivatives, polysaccharides from microbiological sources, arabinogalactanes, galactomannans, dextrans.

3. Pharmaceutical composition according to claims 1 or 2, **characterized in that** the crystallization inhibitors is added in an amount from 0.0001 g to 5.0 g for 1 g of donepezil hydrochloride.

4. A process for the preparation of a pharmaceutical composition according to any of the claims 1 to 3 comprising the steps:
a) dispersing donepezil in a pharmaceutically acceptable solvent and adding a hydrochloric acid solution to the dispersion, or *vice versa,* adding donepezil to a hydrochloric acid solution in a pharmaceutically acceptable solvent, and
b) loading the obtained solution onto a mixture of pharmaceutically acceptable carriers, wherein
a crystallization inhibitor is added to the solution obtained under a) or to the mixture of pharmaceutically acceptable carriers under b).

5. A process according to claim 4, **characterized in that** the molar ratio of donepezil to hydrochloric acid is 1 to 0.5-5.

6. A process according to claim 4, **characterized in that** pharmaceutically acceptable solvents are water, acetone, alcohols, a mixture of water with water-miscible solvents.

7. A process for the preparation of an amorphous form of donepezil hydrochloride, **characterized in that** donepezil hydrochloride is dissolved in a pharmaceutically acceptable solvent or donepezil hydrochloride is prepared *in situ*, a crystallization inhibitor is dissolved in the solution of donepezil hydrochloride and the obtained solution is spray-dried.

8. A use of donepezil hydrochloride obtainable according to the process of claim 7 for the preparation of a pharmaceutical composition, **characterized in that** donepezil hydrochloride is dissolved in a pharmaceutically acceptable solvent and the obtained solution is loaded onto a mixture of pharmaceutically acceptable carriers or that donepezyl hydrochloride is directly loaded onto the pharmaceutical composition.

9. Process for preparing a pharmaceutical composition containing donepezil hydrochloride by dissolving donepezil hydrochloride in a pharmaceutically acceptable solvent and loading the obtained solution onto a mixture of pharmaceutically acceptable carriers or by admixing dry donepezil hydrochloride to pharmaceutically acceptable excipients, wherein the donepezil hydrochloride is obtainable by a process according to claim 7.

## Patentansprüche

1. Pharmazeutische Zusammensetzung, die Donepezilhydrochlorid aufweist und eine amorphe Form oder stabilisierte amorphe Form desselben enthält, **dadurch gekennzeichnet, dass** sie durch Herstellung von Donepezilhydrochlorid *in situ* und Zugabe eines Kristallisationsinhibitors erhältlich ist.

2. Pharmazeutische Zusammensetzung nach Anspruch 1, **dadurch gekennzeichnet, dass** der Kristallisationsinhibitor ausgewählt ist aus mindestens einem von Cellulosederivaten, Polyvinylpyrrolidon und Derivaten davon, Xanthanen, Pektinen, Alginaten, Tragakanth und Derivaten, Gummi arabicum und Derivaten, Carrageenen, Agar und Derivaten, Polysacchariden aus mikrobiellen Quellen, Arabinogalactanen, Galactomannanen, Dextranen.

3. Pharmazeutische Zusammensetzung nach den Ansprüchen 1 oder 2, **dadurch gekennzeichnet, dass** der Kristallisationsinhibitor in einer Menge von 0,0001 g bis 5,0 g auf 1 g Donepezilhydrochlorid zugegegeben wird.

4. Verfahren zur Herstellung einer pharmazeutischen Zusammensetzung nach einem der Ansprüche 1 bis 3, bei dem in den folgenden Stufen
a) Donepezil in einem pharmazeutisch annehmbaren Lösungsmittel dispergiert wird und eine Salzsäurelösung zu der Dispersion gegeben wird,
oder umgekehrt Donepezil zu einer Salzsäurelösung in einem pharmazeutisch annehmbaren Lösungsmittel gegeben wird, und
b) die erhaltene Lösung auf eine Mischung pharmazeutisch annehmbarer Träger gebracht wird, wobei
der unter a) erhaltenen Lösung oder der unter b) erhaltenen Mischung von pharmazeutisch annehmbaren Trägern ein Kristallisationsinhibitor zugefügt wird.

5. Verfahren nach Anspruch 4, **dadurch gekennzeichnet, dass** das Molverhältnis von Donepezil zu Salzsäure 1 zu 0,5 - 5 beträgt.

6. Verfahren nach Anspruch 4, **dadurch gekennzeichnet, dass** die pharmazeutisch annehmbaren Lösungsmittel Wasser, Aceton, Alkohole, eine Mischung von Wasser mit wassermischbaren Lösungsmittel sind.

7. Verfahren zur Herstellung einer amorphen Form von Donepezilhydrochlorid, **dadurch gekennzeichnet, dass** Donepezilhydrochlorid in einem pharmazeutisch annehmbaren Lösungsmittel gelöst wird oder Donepezilhydrochlorid in situ hergestellt wird, ein Kristallisationsinhibitor in der Lösung von Donepezilhydrochlorid gelöst wird, und die erhaltene Lösung sprühgetrocknet wird.

8. Verwendung von Donepezilhydrochlorid, das nach dem Verfahren gemäß Anspruch 7 erhältlich ist, zur Herstellung einer pharmazeutischen Zusammensetzung, **dadurch gekennzeichnet, dass** Donepezilhydrochlorid in einem pharmazeutisch annehmbaren Lösungsmittel gelöst wird und die erhaltene Lösung auf eine Mischung von pharmazeutisch annehmbaren Trägern gebracht wird, oder das Donepezilhydrochlorid direkt auf die pharmazeutische Zusammensetzung gebracht wird.

9. Verfahren zur Herstellung einer pharmazeutischen Zusammensetzung, die Donepezilhydrochlorid enthält, indem Donepezilhydrochlorid in einem pharmazeutisch annehmbaren Lösungsmittel gelöst wird und die erhaltene Lösung auf eine Mischung von pharmazeutisch annehmbaren Trägern gebracht wird, oder indem trockenes Donepezilhydrochlorid mit pharmazeutisch annehmbaren Hilfsstoffen gemischt wird, wobei das Donepezilhydrochlorid nach einem Verfahren gemäß Anspruch 7 erhältlich ist.

## Revendications

1. Composition pharmaceutique de chlorhydrate de donépézil qui en contient une forme amorphe ou une forme amorphe stabilisée, **caractérisée en ce qu'**on peut l'obtenir en préparant *in situ* du chlorhydrate de donépézil et en y ajoutant un inhibiteur de cristallisation.

2. Composition pharmaceutique conforme à la revendication 1, **caractérisée en ce que** l'inhibiteur de cristallisation est choisi comme étant au moins l'un des suivants : dérivés de cellulose, poly(vinyl-pyrrolidone) et ses dérivés, gommes xanthane, pectines, alginates, gomme adragante et ses dérivés, carraghénanes, agar-agar et ses dérivés, polysaccharides d'origine microbienne, arabino-galactanes, galacto-mannanes et dextranes.

3. Composition pharmaceutique conforme à la revendication 1 ou 2, **caractérisée en ce que** l'inhibiteur de cristallisation est ajouté en une proportion de 0,0001 à 5,0 g pour 1 g de chlorhydrate de donépézil.

4. Procédé de préparation d'une composition pharmaceutique conforme à l'une des revendications 1 à 3, qui comporte les étapes suivantes :
a) disperser du donépézil dans un solvant pharmacologiquement admissible et ajouter à la dispersion une solution d'acide chlorhydrique ; ou vice-versa, ajouter du dénépézil à une solution d'acide chlorhydrique dans un solvant pharmacologiquement admissible ;
b) et charger la solution ainsi obtenue sur un mélange de supports pharmacologiquement admissibles ;
et dans lequel on ajoute un inhibiteur de cristallisation à la solution obtenue lors de l'étape (a) ou au mélange de supports pharmacologiquement admissibles utilisé dans l'étape (b).

5. Procédé conforme à la revendication 4, **caractérisé en ce que** le rapport molaire du donépézil à l'acide chlorhydrique vaut de 1/0,5 à 1/5.

6. Procédé conforme à la revendication 4, **caractérisé en ce que** le solvant pharmacologiquement admissible est de l'eau, de l'acétone, un alcool ou un mélange d'eau et d'un solvant miscible à l'eau.

7. Procédé de préparation d'une forme amorphe de chlorhydrate de donépézil, **caractérisé en ce que** l'on dissout du chlorhydrate de donépézil dans un solvant pharmacologiquement admissible ou l'on prépare *in situ* du chlorhydrate de donépézil, on dissout un inhibiteur de cristallisation dans la solution de chlorhydrate de donépézil, et l'on fait sécher la solution ainsi obtenue par pulvérisation.

8. Utilisation de chlorhydrate de donépézil accessible par un procédé conforme à la revendication 7 pour la préparation d'une composition pharmaceutique, **caractérisée en ce que** l'on dissout du chlorhydrate de donépézil dans un solvant pharmacologiquement admissible et l'on charge la solution ainsi obtenue sur un mélange de supports pharmacologiquement admissibles, ou **en ce que** l'on charge directement du chlorhydrate de donépézil dans la composition pharmaceutique.

9. Procédé de préparation d'une composition pharmaceutique contenant du chlorhydrate de donépézil par dissolution de chlorhydrate de donépézil dans un solvant pharmacologiquement admissible et chargement de la solution ainsi obtenue sur un mélange de supports pharmacologiquement admissibles ou par addition et mélange de chlorhydrate de donépézil sec à des excipients pharmacologiquement admissibles, dans lequel le chlorhydrate de donépézil est accessible par un procédé conforme à la revendication 7.
